# EUROPEAN PATENT SPECIFICATION

(11) **EP 1 902 719 B1**
(45) Date of publication and mention of the grant of the patent: **16.02.2011**
(21) Application number: 06757952.4
(22) Date of filing: 17.05.2006
(51) Int. Cl.: A61K 35/74, A61K 38/43, A61P 31/04

(54) **AGENT EXHIBITING BACTERICIDAL ACTION WITH RESPECT TO VEGETATIVE AND SPORE CELLS BACILLUS ANTHRACIS AND ITS USE FOR ANTHRAX PREVENTION AND TREATIMENT**
MITTEL MIT BAKTERIZIDER WIRKUNG GEGEN VEGETATIVE UND SPORENZELLEN VON BACILLUS ANTHRACIS UND DESSEN VERWENDUNG ZUR PRÄVENTION UND BEHANDLUNG VON ANTHRAX
PRODUIT POSSEDANT UNE ACTION BACTERICIDE VIS-A-VIS DES CELLULES VEGETATIVES ET CRYPTOGAMES DE BACILLUS ANTHRACIS, PROCEDE DE PREVENTION ET DE TRAITEMENT DE L'ANTHRAX

(30) Priority: 18.05.2005 RU 2005114964
(43) Date of publication of application: 26.03.2008
(73) Proprietor: Uchrezhdenie Rossiiskoi Akademii Nauk Institut Biokhimii i Fisiologii Mikroorganismov Im. G.K. Skrjabina Ran (IBFM RAN), Puschino, Moskovskaja obl. 142290 (RU)
(72) Inventor: SCHISCHKOVA, Nina Andreevna, Serpukhovsky raion, Moskovskaya 142279 (RU); STARIZIN, Nikolai Andreevich, Serpukhovsky raion, Moskovskaya 142279 (RU); MARININ, Leonid Ivanovicn, Serpukhovsky raion, Moskovskaya 142279 (RU); STEPNAIA, Olga Andreevna, Puschino, Moskovskaya obl., 142290 (RU); KULAEV, Igor Stepanovich, Moscow, 117296 (RU); ZFASMAN, Irina Matveevna, Puschino, Moskovskaya obl. 142290 (RU); BEGUNOVA, Elena Albertovna, Puschino, Moskovskaya obl. 142290 (RU); CHAIKA, Irina Alexandrovna, Voronezh, 394029 (RU)
(74) Representative: HOFFMANN EITLE
(86) International application number: PCT/RU2006/000242
(87) International publication number: WO 2006/123969

(56) References cited:
- RU-C1- 2 111 744
- RU-C1- 2 122 026
- RU-C1- 2 216 349
- RU-C2- 2 193 063
- US-A1- 2003 148 497
- US-A1- 2004 014 707
- US-A1- 2005 004 030
- MURANOVA T A ET AL: "Structural investigations and identification of the extracellular bacteriolytic endopeptidase L1 from Lysobacter sp. XL1." BIOCHEMISTRY. BIOKHIMII A MAY 2004, vol. 69, no. 5, May 2004 (2004-05), pages 501-505, XP009129305 ISSN: 0006-2979
- SCHUCH RAYMOND ET AL: "A bacteriolytic agent that detects and kills Bacillus anthracis." NATURE 22 AUG 2002, vol. 418, no. 6900, 22 August 2002 (2002-08-22), pages 884-889, XP002903133 ISSN: 0028-0836

## Description

### Field of the Invention

The invention refers to the field of biotechnology, medicine, and veterinary and can be used for anthrax prevention and treatment.

### Background of the Invention

Anthrax is prevented either by vaccination or by prolonged antibiotic therapy.

### 1. Vaccination

It is known that at present people are immunized against anthrax either with live anthrax vaccine based on attenuated strain or with inactivated adsorbed anthrax vaccine based on protective antigen. In the recent years, the studies have been performed to develop novel genetically engineered vaccines based on the recombinant lethal toxin of *B. anthracis* (Price B.M., Liner A.L., Park S., Leppla S.H., Mateczun A., Galloway D.R. Protection against anthrax lethal toxin challenge by genetic immunization with a plasmid encoding the lethal factor protein. 2001, Infect Immun, N 69, pp.4509-4515).

The only vaccine used for immunization of people against anthrax in the USA and West Europe is inactivated adsorbed anthrax vaccine (BioPort Corporation, Lancing, Michigan, USA). It is a cell-free filtrate of *B. anthracis* culture grown in a liquid nutrient medium. The vaccine is produced using a toxigenic strain which does not form a capsule and is known as V770-NR1-R. The filtrate contains a mixture of life activity products of *B. anthracis* cells, including protective antigen (Turnbull P.S.B., Broster M.G., Carman J.A., Manchee R.J., Melling J. Development of antibodies to protective antigen and lethal factor components of anthrax toxin in humans and guinea pigs and their relevance to protective immunity. 1986, Infect. Immun., N 52, pp. 356-363). Vaccine components are adsorbed on aluminum hydroxide, which is used as an adjuvant. The efficiency and safety of the vaccine have been certified by the USA Food and Drug Administration (FDA).

In Russia, people of the anthrax infection risk group (workers of factories for live-stock products processing) are systematically immunized with live attenuated anthrax vaccine STI. Risk groups are liable to annual vaccination; emergency vaccinations take place as needed, in parallel with preventive antibiotic therapy, because introduction of the vaccine without effective antibiotics does not prevent disease development.

### 2. Antibiotic therapy

Anthrax prevention includes a long-term (60-100 days) antibiotic therapy. Preparations such as doxycycline (Centers for Disease Control and Prevention. Update: Investigation of Bioterrorism-Related Anthrax and Interim Guidelines for Exposure Management and Antimicrobial Therapy. MMWR Morb Mortal Wkly Rep, 2001, October 24; N 50, pp. 909-919) and fluoroquinolones (cyprofloxacin, levofloxacin, moxyfloxacin, gatifloxacin) (Ferrandiz M.J., Fenoll A., Linares J., De La Campa A. Horizontal transfer of parC and gyrA fluoroquinolone-resistant clinical isolates of Streptococcus pneumoniae. 2000, Antimicrob Agent Chemother, N 44, pp. 840-847), are used most frequently. For children, pregnant women and nursing mothers, the optimal alternative is amoxicillin (Prophylactic treatment of anthrax with antibiotics editorial. 2001, BMJ, N 323, pp. 1017-1018; Centers for Disease Control and Prevention. Updated recommendations for antimicrobial prophylaxis among asymptomatic pregnant women after exposure to Bacillus anthracis. 2001, MMWR Morb Mortal Wkly Rep., N 50, p.960) which allows avoiding the development of toxic effects typical of fluoroquinolones and tetracyclines. At the same time, FDA has approved penicillin G procaine to be used as a target preventive therapy in all age groups (Food and Drug Administration. Prescription drug products: doxycycline and penicillin G procaine administration for inhalational anthrax (post-exposure). Fed. Reg, 2001; N66, pp. 55679-55682).

Concurrent application of antibiotics and vaccine is considered to be most preferable for emergency prevention of anthrax (Friedlander A.M., Welkos S.L., Pitt M.L.M., Ezzell J.W., Worsham P.L., et al. 1993, Postexposure prophylaxis against experimental anthrax. J Infect Dis., N 167, pp. 1239-1243.; Henry L. Inhalational Anthrax: Threat, Clinical Presentation, and Treatment. 2001, J Am. Acad. Nurse Pract., N 13, pp. 164-168).

However, the known methods of prevention have disadvantages. Disadvantages of vaccination:
1. Allergic reactions: edema and pain at the point of vaccine introduction, infiltration, headache, arthralgia, asthenia, and skin itch. In rare cases: the so-called delayed or late reactions shown by dyspnea, excessive sweating, skin pallor, and urticarial exanthema (the data of the System of registration of unwanted reactions associated with vaccination **(VAERS - *Vaccine Adverse Event Reporting System*)**).
2. The *B. anthracis* infection in anamnesis is a contraindication to vaccination.
3. The live attenuated anthrax vaccine STI, which is used in Russia for immunization of people from the risk group (workers of factories for live-stock products processing) causes local and general reactions.

### Disadvantages of antibiotic therapy:

1. Long-term application of antibiotics may result in emergence of antibiotic-resistant strains of the anthrax pathogen. The anthrax pathogen may acquire resistance to penicillins, tetracyclines (doxycycline), sulfamethoxazole, trimetoprim, trimoxazol, aztreonam, and other antibiotics.
   Some cases are known, when *B anthracis* strains developed resistance at cultivation on the media containing sub-inhibiting concentrations of some antibiotics (doxycycline, erythromycin, azythromycin, claritromycin, ciprofloxacin, alatrofloxacin, gatifloxacin). In one of the studies, several passages of the pathogen on a medium with any fluoroquinolone resulted in MIC increase for all antibiotics of this group.
2. Long-term therapy with antimicrobial preparations facilitates the selection of pathogenic microorganisms which are resistant to them as well as other more widespread and clinically significant pathogens. Prolonged application of antibiotics contributes to the formation of a novel population of pneumococci, resistant both to penicillin and to fluoroquinolones; besides, it leads to the death of normal microflora in human organism.
3. In the course of preventive antimicrobial therapy, patients may have unwanted side reactions such as nausea, vomit, diarrhea and stomach ache, syncope, vertigo, heartburn, rash of different nature, nettle rush and skin itching, as well as the symptoms probably associated with anaphylactic reactions (difficult breathing, aglutition, sense of constraint in the neck, edema of lips, tongue, or face).
4. Long-term application of ciprofloxacin in people below 16 may result in development of arthropathy; doxycycline disturbs the growth of bones in children and, besides, is hepatotoxic.
5. Long-term preventive antibiotic therapy weakens immune system and induces secondary mycoses.

Some methods of treatment for anthrax with antimicrobial preparations are known.

Native *B. anthracis* strains, including those isolated in the USA in the autumn of 2001, are sensitive to many antibiotics, e.g., penicillin, amoxicillin, doxycycline, tetracycline, claritromycin, clyndamycin, riphampicin, vancomycin, chloramphenicol, and ciprofloxacin. According to the data of *in vitro* studies, cefazolin and other cefalosporines of I generation are also active to *B. anthracis.* Eleven strains of the anthrax pathogen isolated in the USA in the autumn of 2001 are moderately resistant to erythromycin and azythromycin. Taking into account the experience in treating patients with the inhalation form of anthrax involved in the infective episode in the USA in the autumn of 2001, CDC (Center for Drug Control) recommends the combination of antimicrobial preparations as a starting therapy for patients with the inhalation form of the disease, as well as for patients with systemic manifestations of the disease (Hanter P. In response to Recent Events: Update on Bacillus anthracis. 2001, ESCMID News, N3, pp. 23-26).

The combined antibacterial therapy usually includes ciprofloxacin or doxycycline as an agent of choice in combination with one of the antibiotics, to which the native *B. anthracis* strains are sensitive (by the results of *in vitro* studies and experiments on model animals).

The duration of antibacterial therapy for skin, pneumonic, and gastrointestinal forms of anthrax at inhalation contagion process is 60 to 100 days (Centers for Disease Control and Prevention. Update: Investigation of Bioterrorism-Related Anthrax and Interim Guidelines for Exposure Management and Antimicrobial Therapy. 2001, MMWR Morb. Mortal. Wkly Rep. October 24; N50, pp. 909-919). Long-term application of antimicrobial preparations is conditioned by the phenomenon of "delayed spore germination" of *B. anthracis.*

The essence of the above phenomenon is that the inhaled spores of the anthrax pathogen can persist in inactive state in alveoli for a long time (up to several weeks) until captured by alveolar macrophages. Only then they start to germinate into vegetative forms and propagate.

There is a method of treatment with immune serum obtained from vaccinated people, which contains the antibodies, mainly IgG, to the anthrax pathogen toxin factors. However, this method cannot be considered prospective due to the risk of infection with the viruses of hepatitis C, hepatitis B, HIV, and other pathogens.

Other methods of anti-anthrax therapy also have disadvantages.

The antimicrobial preparations used for treating anthrax are active only against germinating spores and propagating vegetative forms but have no effect on the mature spores of *B. anthracis.* On premature termination of antibiotic therapy, the survived pathogen spores may cause recurrence, provided that their quantity is high enough to overcome the host defenses of a macroorganism. However, it should be noted that this phenomenon has never been observed at skin and gastrointestinal forms of anthrax.

Besides, prescription of antibacterial drugs is known to extend the period of incubation.

It is also known that the selection of *B. anthracis* strains resistant to different antimicrobial preparations is possible.

There are widely known cases of development by *B. anthracis* strains of resistance to doxycycline, erythromycin, azythromycin, claritromycin, ciprofloxacin, alatrofloxacin, gatifloxacin, benzylpenicillin, and penicillin.

Besides, unwanted drug reactions and complications may occur in the course of treatment.

### Detailed Description of Preferred Embodiments of the Invention

The objective of invention is development of the means and methods of anthrax prevention and treatment.

The technical effect of proposed methods is as follows:
- Reduction of the periods of prevention of and treatment for anthrax caused by *Bacillus anthracis* strains, including antibiotic-resistant ones
- Absence of the main side effects
- Minimization of the risk of recurrence

The essence of invention consists in application of a bacteriolytic complex produced by bacterial strain *Lysobacter sp.* XL 1 as a remedy that has a bactericidal effect on vegetative and spore cells of *Bacillus anthracis* causing anthrax.

The essence of the method of anthrax prevention is that the bacteriolytic complex produced by bacterial strain *Lysobacter sp.* XL 1 is used as acting substance, through injections of the preparation, application of bandages or tissue papers sodden with the acting substance, or treatment of probable infection focus with aerosol.

The essence of anthrax treatment is that the bacteriolytic complex produced by bacterial strain *Lysobacter sp.* XL 1 is used as acting substance, through injections of the acting substance for generalized forms of anthrax, encompassing injections or aerosol treatment of lesion focus for the skin form.

The experimental anthrax is treated and prevented with the bacteriolytic complex solution in phosphate buffer, which is prepared immediately before application. Initial concentration of the solution is 10 mg/ml. Animals are injected with 0.1 ml of the bacteriolytic complex solution, i.e., the dose is 1 mg/mouse.

The bacteriolytic complex solution is introduced subcutaneously into the inner surface of femur of the extremity infected by the anthrax pathogen. Anthrax is prevented and treated by single introduction of the bacteriolytic complex no later than three hours after the infection (prevention) or no later than 24 h (treatment).

The bacteriolytic complex includes the following components, mass%:
Bacteriolytic enzymes (muramidase, muramoylalanine amidase, endopeptidase, bacteriolytic enzyme ∼ 22 kDa)...2-5
Protease...1-2
Ballast components...2-4
Polysaccharide...the rest
[International Application WO 02/44352, C12N 9/00, 2002, or patent RU N2 2193063, C12N 9/00, 2002].

In the above materials, the strain-producer of the bacteriolytic complex is named as *Xanthomonas campestris X1 L.* At the same time, it is mentioned in the International Application that the identification according to Bergey's Manual (Bergey's Manual of Systematic Bacteriology. Eds. Noel R. Krieg, John G. Holt. Baltimore; London, Williams & Wilkins. 1984, v.1) permits affiliation of this bacterium with the species *Xanthomonas campestris* or the genus *Lysobacter.*

On determination of the nucleotide sequence of the gene encoding 16-S RNA of the bacterium and analysis of this sequence using the software for DNA and RNA analysis from "Ribosomal Database Project" (http: //rdp. cme. msu. edu/html/), the strain was referred to the genus *Lysobacter sp. XL1.*

The authors of proposed invention have demonstrated that the bacteriolytic complex lyzes vegetative cells of gram-positive bacteria, in particular, *Bacillus anthracis, Bacillus cereus* 217, *Bacillus subtilis* W23, *Bacillus subtilis* 168, and has a sporicidal effect on mature spores of the above microorganisms.

The method of prevention and treatment is as follows: the bacteriolytic complex produced by bacterial strain *Lysobacter sp. XL1* is introduced as subcutaneous injections into infected animals.

The possibility of realization of the method is supported by, but not limited to, the following examples.

### Example 1. Lysis of B. anthracis vegetative cells

Spore suspension of *B. anthracis* strain STI-1 is introduced into a flask with L-broth to a final concentration of 10⁶-10⁷ spores/ml. The flask in incubated at 37°C for 18 h. Cells are concentrated by centrifugation, washed with physiological solution, and then suspended in 0.01 M phosphate buffer, pH 8.0-8.5, so that the final concentration of vegetative cells of the anthrax pathogen would be ∼ 10⁸ cells/ml by the turbidity standard of State Institute of Standardization and Control of Biopreparations: 10 units. Cell suspension is poured into similar biological test tubes by 5 ml, with fresh solution of the bacteriolytic complex in the final concentration of 50-2500 mkg/ml added into each tube. The tubes are incubated at 37°C, and the turbidity of experimental samples is monitored visually as compared with the control sample in equal periods of time.

The data presented in Table 1 lead to a conclusion that the bacteriolytic complex in a concentration of 50-2500 µg/ml has a lyzing effect on vegetative cells of *B. anthracis.*

### Example 2. Sporicidal effect of the bacteriolytic complex on mature spores of B. cereus 217, B. subtilis 168, W23, and B. anthracis STI-1.

The bacteriolytic complex, 2.5 mg, is added to 2 ml of mature spores in 10 mM Tris-HCl buffer, pH 8.0 (OD₆₀₀∼ 0.02), and incubated on a shaker at 37°C for 8 h. Then another 2.5 mg of the bacteriolytic complex is added to the experimental test tube and incubated for 12 h on a shaker at 37°C. In the control, 10 mM Tris-HCl buffer, pH 8.0, is added instead of the enzyme. Spores are washed 2 times with 0.1 M NaCl and 2 times with water. For activation of spore germination, inactivation of the enzyme (bacteriolytic complex), and disturbance of viability of vegetative cells, the mixture is heated at 85°C for 20 min and then transferred to 5 ml of liquid LB medium for germination. Germination proceeds at 37°C. Inoculations from the liquid medium to plates with solid nutrient medium are made in 0, 1.5, 3, 7, 9, and 24 h. The growth is controlled by OD₆₀₀ in the liquid medium and visually on the plates.

The results show that the bacteriolytic complex inhibits the viability of 100% of mature spores of *B. subtilis* and *B. cereus* and 85% of mature spores of *B. anthracis.*

### Example 3. The effect of the bacteriolytic complex on germinating spores of B. anthracis STI-1.

Spore suspension in water is prepared in the concentration of 3 × 10⁸ spores/ml. Spores are activated by heating at 70°C for 30 min and then inoculated in a flask with L-broth with the initial optical density of 0.1. Samples are taken from the flask in 30, 60, and 120 min. Probe samples (5 ml) are precipitated, washed with water, and re-suspended in the working buffer for the enzyme containing 1 mg of the bacteriolytic complex/ml of 10 mM Tris-HCl buffer, pH 8.0. Samples are incubated in a thermostat at 37°C for 18 h. On the end of incubation, spores are centrifuged, twice washed with water, tittered in physiological solution, and inoculated on plates with L-agar to determine the quantity of germinated spores.

The data from Table 2 show that the bacteriolytic complex in the concentration of 1 mg/ml has a bactericidal effect on germinating spores of the anthrax pathogen, and the intensity of this effect depends on the stage of spore germination.

Example 4. The method of treatment for experimental anthrax infection using the bacteriolytic complex.

The experiment is performed in outbred white mice, 18-20 g. Fifty mice are injected subcutaneously with 5 × 10² spores of strain *Bacillus anthracis* 71/12 (Tsenkovsky vaccine II) per mouse and then are divided into 10 experimental groups of 5 mice each (Table 3). Experimental mice are injected subcutaneously with drugs.

The mice are observed during 14 days.

The control group as well as groups 8 and 9, where mice are treated with bovine serum albumin (1 mg/mouse), show 100% mortality of experimental animals on day 6 after the contagion. In group 10 (treatment with lyzozyme), the mortality is 80%. When mice are treated with the bacteriolytic complex in the concentration of 0.1 mg/mouse (groups 5 and 6), the survival is 80% and 60%, respectively. Introduction of the preparation into uninfected pad (groups 4 and 7) proves to be inefficient: the survival is 20% and 0%, respectively. When the bacteriolytic complex in the concentration of 1 mg/mouse (groups 2 and 3) is injected into the infected extremity of experimental animals, survival is 100%.

Example 5. The method of treatment for pneumonic form of anthrax using the bacteriolytic complex.

To estimate the therapeutic effect of the bacteriolytic complex at pneumonic form of anthrax, 10 mkl of *Bacillus anthracis* spore suspension containing 10⁹ spores/ml of strain 71/12 (Tsenkovsky vaccine II) is introduced intranasally to outbred white mice. The bacteriolytic complex is introduced intranasally as 1 mg of lysoamidase in 20 mkl of buffer (Table 4, groups 2 and 3) and subcutaneously as 1 mg/mouse (Table 4, group 4). The mice are observed during 14 days. As follows from Table 4, the mortality of infected animals is 100% without the treatment with the bacteriolytic complex (group 1). The treatment for pneumonic form of anthrax shows the high efficiency of subcutaneous introduction of the bacteriolytic complex (60% survival in group 5) as compared with intranasal introduction (20% survival in groups 3 and 4).

### Example 6. The method of treatment for anthrax by the bacteriolytic complex at intraperitoneal introduction of Bacillus anthracis spores.

The experiment is performed by the following scheme:
Spores of the anthrax pathogen, strain 71/12 (Tsenkovsky vaccine II), in a dose of 1.2 × 10³ spores are introduced intraperitoneally to outbred white mice. Then the animals are divided into three groups: group 1 (untreated animals); group 2 (intraperitoneal introduction of the bacteriolytic complex solution); and group 3 (treatment with the bacteriolytic complex solution through its subcutaneous introduction into the inner part of femur. The therapeutic effect of 90% and 100%, respectively, is observed against the death of control animals (Table 5).

### Example 7. The method of treatment of animals infected by vegetative cells of Bacillus anthracis with the bacteriolytic complex.

Vegetative cells of strain *Bacillus anthracis* STI-1 grown on L-agar are injected subcutaneously into the inner femur surface of outbred white mice: 10⁸ cells/mouse. The bacteriolutic complex solution is injected subcutaneously into the same place in 1 h.

Untreated mice die in 24 h in 100% of cases, while the mortality in the group of treated animals is only 10%.

### Example 8. The method of treatment for anthrax caused by Bacillus anthracis strain STI-PRTS (resistant to rifampicin, ampicillin, tetracycline, and doxycycline) with the bacteriolytic complex.

### Mice of CBA line are infected subcutaneously with spore suspensions of strains STI-1 and STI-PRTS. The strains are characterized in Table 6.

Contaminating doses are 10⁷, 10⁶, and 10⁵ spores for both strains. Animals are treated with doxycyclin or bacteriolytic complex. Doxycyclin is introduced per os, 100 mkg/mouse; doxycyclin therapy is started in 3 h after the infection, with introduction of the preparation once a day during a week. The bacteriolytic complex is introduced subcutaneously into the inner femur surface of the infected pad, 1 mg/mouse; the treatment is carried out once in 3 h after the infection (Table 7).

The therapeutic effect of doxycyclin and bacteriolytic complex in the treatment of mice infected by spores of *Bacillus anthracis* STI-1 (initial) has been shown to depend on infection dose. Application of the bacteriolytic comeplx as a drug in the treatment of mice infected by strain *Bacillus anthracis* STI-PRTS results in 100% survival of animals at infection doses of 10⁵ and 10⁶ spores, whereas the doxycyclin therapy of animals infected by the same strain was inefficient at all infection doses.

Efficiency of the preparation was assessed in conclusive experiments at infection of golden hamsters with a virulent strain of *Bacillus anthracis.* Animals had subcutaneous injections of strain Ch-7 spore suspension, 100 LD₅₀, into the inner femur surface. The first group of animals has subcutaneous introduction of the bacteriolytic complex, 5 mg/hamster, in 3 hours into the infected pad. The second group of animals receives the preparation in the same dose in 3 and 24 h into the same pad. The third group of animals receives the preparation in the same dose in 3 and 24 h into another pad. The animals are observed during 14 days.

The analysis of the data from Table 8 shows that the preparation efficiency in treatment and prevention is 60-100% at different regimes of introduction and depends on the place of application.

The above estimates of the efficiency of preparation *in vitro* against the anthrax pathogen and the data on the efficiency of therapy for acute anthrax infection proceeding with the infection-toxic shock in experimental animals lead to a conclusion that the preparation can be used for anthrax treatment in people and livestock.

### Industrial Applicability

Thus, we propose:
- a drug having bactericidal effect on vegetative and spore cells of *Bacillus anthracis* bacteria;
- a novel method of anthrax prevention and treatment which provides:
   (a) reduction of the periods of prevention of and treatment for anthrax caused by *Bacillus anthracis* strains, including antibiotic-resistant ones;
   (b) absence of major side effects;
   (c) minimization of the risk of recurrence

Proposed inventions can be used in medicine and veterinary for anthrax prevention and treatment.

**Table 1**

| ***Lysis of* B. anthracis *vegetative cells in the presence of bacteriolytic complex*** | | | | | | |
|---|---|---|---|---|---|---|
| Strain | Concentration of bacteriolytic complex, µg/ml | | | | | |
| | 50 | 100 | 250 | 500 | 1000 | 2500 |
| STI-1 | + | + | + | + | + | + |

**Table 2**

| Number of *B. anthracis* colonies on L-agar plates | | | |
|---|---|---|---|
| Time of sample taking from L-broth | Number of colonies grown at inoculation of sample dilution | | |
| | 3 | 4 | 5 |
| 0 | Confluent growth | Confluent growth | 300 |
| 30 min | Confluent growth | Confluent growth | 120 |
| 1 h | 270, 250, 220 | 28, 34, 32 | 0 |
| 2h | 5,4,7 | 1, 2, 1 | 0 |

**Table 3**

| Efficiency of the treatment for experimental anthrax with bacteriolytic complex | | | | | | |
|---|---|---|---|---|---|---|
| Group of animals | Number of animals | Death of animal on day ... | | | | Number of perished/ Number of infected |
| | | 2 | 3 | 6 | 7 | |
| 1 - infecting dose control | 5 | 2 | 1 | 2 | | 5/5 |
| 2 - treatment with bacteriolytic complex, 1 mg/mouse, in 3 h | 5 | 0 | 0 | 0 | 0 | 0/5 |
| 3 - treatment with bacteriolytic complex, 1 mg/mouse, in 24 h | 5 | 0 | 0 | 0 | 0 | 0/5 |
| 4 - treatment with bacteriolytic complex, 1 mg/mouse, in 24 h into other pad | 5 | 0 | 3 | 0 | 1 | 4/5 |
| 5 - treatment with bacteriolytic complex, 0.1 mg/mouse, in 3 h | 5 | 1 | 0 | 0 | 0 | 1/5 |
| 6 - treatment with bacteriolytic complex, 0.1 mg/mouse, in 24 h | 5 | 1 | 0 | 0 | 1 | 2/5 |
| 7 - treatment with bacteriolytic complex, 0.1 mg/mouse, in 24 h into other pad | 5 | 1 | 2 | 2 | 0 | 5/5 |
| 8 - treatment with bovine serum albumin, 1 mg/mouse, in 3 h | 5 | 2 | 1 | 1 | 1 | 5/5 |
| 9 - treatment with bovine serum albumin, 1 mg/mouse, in 24 h | 5 | 1 | 2 | 2 | 0 | 5/5 |
| 10 - treatment with lyzozyme, 1 mg/mouse, in 3 h | 5 | 1 | 3 | 0 | 0 | 4/5 |

**Table 4**

| Efficiency of the treatment for pneumonic form of anthrax with bacteriolytic complex | | | | | | |
|---|---|---|---|---|---|---|
| Groups of animals | Number of animals in group | Death of animals on day ... | | | | Number of perished/ Number of infected |
| | | 4 | 5 | 6 | 7 | |
| I - Infecting dose control | 5 | 4 | 1 | | | 5/5 |
| II - Intranasal introduction of bacteriolytic complex, 1 mg/mouse, in 3 h | 5 | 4 | 0 | 0 | 0 | 4/5 |
| III - Intranasal introduction of bacteriolytic complex, 1 mg/mouse, in 24 h | 5 | 4 | 0 | 0 | 0 | 4/5 |
| IV - Subcutaneous introduction of bacteriolytic complex into pad, 1 mg/mouse, in 3 and 24 h | 5 | 1 | 1 | 0 | 0 | 2/5 |
| V - Intranasal introduction of bacteriolytic complex, 1 mg/mouse, without culture | 5 | 0 | 0 | 0 | 0 | 0/5 |

**Table 5**

| Efficiency of the treatment for anthrax at intraperitoneal infection of mice | | | | | | |
|---|---|---|---|---|---|---|
| Groups of animals | Number of animals in group | Death of animals on day ... | | | | Number of perished/ Number of infected |
| | | 2 | 3 | 4 | 6 | |
| I - Infecting dose control | 10 | 2 | 2 | 4 | 2 | 10/10 |
| II - Intraperitoneal introduction of bacteriolytic complex, I mg/mouse | 10 | 1 | 0 | 0 | 0 | 1/10 |
| III - Subcutaneous introduction of bacteriolytic complex, 1 mg/mouse | 10 | 0 | 0 | 0 | 0 | 0/10 |

**Table 6**

| Estimation of antibiotic resistance of anthrax vaccine strains by disc technique | | | | | | | |
|---|---|---|---|---|---|---|---|
| Strain | Zone of culture growth delay (mm) at using discs with antibiotics | | | | | | |
| | Amp | Rif | Ery | Ole | Km | Cm | Tc |
| STI-1 | 4 | 2 | 3 | 4 | 6 | 4 | 7 |
| STI - PRTS | 0 | 0 | 3 | 4 | 6 | 4 | 0 |

**Table 7**

| Efficiency of the treatment of animals infected with antibiotic-resistant strain *Bacillus anthracis* STI-PRTS | | | |
|---|---|---|---|
| Group of animals Strain | **Death of animals among those taken for the experiment at infection by doses** | | |
| | 10⁷ spores | 10⁶ spores | 10⁵ spores |
| 1. STI-1 - control (no treatment) | 3/3 | 3/3 | 3/3 |
| 2. STI-1 -treatment with doxycycline (7 days) | 3/3 | 1/3 | 0/3 |
| 3. STI-1 -treatment with bacteriolytic complex once in 3 h | 3/3 | 0/3 | 0/3 |
| 4. STI-PRTS - control (no treatment) | 3/3 | 3/3 | 3/3 |
| 5. STI-PRTS -treatment with doxycycline (7 days) | 3/3 | 3/3 | 3/3 |
| 6. STI-PRTS -treatment with bacteriolytic complex once in 3 h | 3/3 | 0/3 | 0/3 |

**Table 8**

| Efficiency of the treatment for anthrax at subcutaneous infection of hamsters by virulent strain Ch-7 | | | | | |
|---|---|---|---|---|---|
| Groups of animals | Number of animals in group | Death of animals on day ... | | | Number of perished/ Number of infected |
| | | 2 | 3 | 4 | |
| I - control (no treatment) | 5 | 2 | 3 | - | 5/5 |
| 2 - treatment with bacteriolytic complex once in 3 h into the same pad | 5 | - | - | - | 0/5 |
| 3 - treatment with bacteriolytic complex in 3 and 24 h into the same pad | 5 | - | - | - | 0/5 |
| 4 - treatment with bacteriolytic complex in 3 and 24 h into other pad | 5 | 1 | - | 1 | 2/5 |

## Claims

1. A bacteriolytic complex produced by bacterial strain Lysobacter sp. XL 1 including the bacteriolytic enzymes muramidase, muramoylalanine amidase, endopeptidase, and bacteriolytic enzyme - 22kDa (2-5 mass%); protease (1-2 mass%); ballast components (2-4 mass%) and polysaccharides (ad 100 mass%) for use in the treatment or prevention of anthrax.

2. The bacteriolytic complex according to claim 1 which is suitable to be injected subcutaneously by single introduction of a bacteriolytic complex solution no later than 3 h after the infection.

3. The bacteriolytic complex according to claim 1 which is suitable to be applied to a supposed place of pathogen penetration by a bandage (tissue paper) sodden with the bacteriolytic complex.

4. The bacteriolytic complex according to claim 1, whereby an aerosol containing the bacteriolytic complex is suitable to treat a probable nidus of infection.

5. The bacteriolytic complex according to claim 1, whereby the bacteriolytic complex is suitable to treat the skin form of anthrax by subcutaneous injections, encompassing injections or aerosol treatment of lesion focus, and to treat the generalized forms by subcutaneous injections.

6. The bacteriolytic complex according to any one of claims 1-5, whereby the bacteriolytic complex is used in the form of solution in phosphate buffer which is prepared immediately before application.

7. The bacteriolytic complex according to claim 2, whereby the bacteriolytic complex solution is suitable to be introduced subcutaneously into the extremity where the nidus of infection is located.

8. The bacteriolytic complex according to claim 1, whereby the bacteriolytic complex is suitable to be introduced no later than 3 h or 24 h after infection.

9. Pharmaceutical composition including a bacteriolytic complex according to any one of claims 1-8 for use in the treatment or prevention of anthrax.

10. Use of a bacteriolytic complex according to any one of claims 1-8 in the manufacture of a medicament for the treatment and prevention of anthrax.

11. The bacteriolytic complex produced by bacterial stain Lysobacter sp. XL 1 according to any one of claims 1-8 for use as a bactericidal agent on vegetative and spore cells of *Bacillus anthracis.*

## Patentansprüche

1. Bakteriolytischer Komplex, hergestellt durch Bakterienstamm Lysobacter sp. XL 1, welcher folgendes einschließt: die bakteriolytischen Enzyme Muramidase, Muramoylalaninamidase, Endopeptidase und bakteriolytisches Enzym 22kDa (2 bis 5 Massen%); Protease (1 bis 2 Massen%); Ballastkomponenten (2 bis 4 Massen%) und Polysaccharide (ad 100 Massen%) zur Verwendung in der Behandlung oder Vorbeugung von Anthrax.

2. Bakteriolytischer Komplex gemäß Anspruch 1, der zur subkutanen Injektion einer einmaligen Eintragung einer bakteriolytischen Komplexlösung nicht später als 3 Stunden nach der Infektion geeignet ist.

3. Bakteriolytischer Komplex gemäß Anspruch 1, der zum Anwenden eines mit dem bakteriolytischen Komplex getränkten Verbandes (Papiertaschentusch) an der mutmaßlichen Stelle des pathogenen Eindringens geeignet ist.

4. Bakteriolytischer Komplex gemäß Anspruch 1, wobei ein den bakteriolytischen Komplex enthaltenes Aerosol geeignet ist einen wahrscheinlichen Infektionsherd zu behandeln.

5. Bakteriolytischer Komplex gemäß Anspruch 1, wobei der bakteriolytische Komplex geeignet ist, die Hautform von Anthrax durch subkutane Injektion, begleitende Injektion und/oder Aerosolbehandlung des Wundherds zu behandeln und die allgemeinen Formen durch subkutane Injektionen zu behandeln.

6. Bakteriolytischer Komplex gemäß irgendeinem der Ansprüche 1 bis 5, wobei der bakteriolytische Komplex in Form einer Phosphatpufferlösung verwendet wird, die unmittelbar vor der Anwendung hergestellt ist.

7. Bakteriolytischer Komplex gemäß Anspruch 2, wobei die bakteriolytische Komplexlösung zur subkutanen Eintragung in die Extremität geeignet ist, bei welcher der Infektionsherd lokalisiert ist.

8. Bakteriolytischer Komplex gemäß Anspruch 1, wobei der bakteriolytische Komplex zur Eintragung nicht später als 3 oder 24 Stunden nach der Infektion geeignet ist.

9. Pharmazeutische Zusammensetzung, die einen bakteriolytischen Komplex gemäß irgendeinem der Ansprüche 1 bis 8 einschließt, zur Verwendung in der Behandlung oder Vorbeugung von Anthrax.

10. Verwendung eines bakteriolytischen Komplexes gemäß irgendeinem der Ansprüche 1 bis 8 zur Herstellung eines Medikaments zur Behandlung und Vorbeugung von Anthrax.

11. Bakteriolytischer Komplex, hergestellt durch Bakteriostamm Lysobacter sp. XL 1 gemäß irgendeinem der Ansprüche 1 bis 8 zur Verwendung als Bakterizid an vegetativen und Sporenzellen des Bacillus anthracis.

## Revendications

1. Complexe bactériolytique produit par une souche bactérienne de *Lysobacter sp. XL 1* comprenant les enzymes bactériolytiques muramidase, muramoylalanine amidase, une endopeptidase, et une enzyme bactériolytique d'environ 22 kDa (2 % à 5 % en masse) ; une protéase (1 % à 2 % en masse) ; des composants de lest (2 % à 4 % en masse) et des polysaccharides (jusqu'à 100 % en masse) destiné à une utilisation dans le traitement ou la prévention de l'anthrax.

2. Complexe bactériolytique selon la revendication 1, qui est approprié pour être injecté par voie sous-cutanée par introduction unique d'une solution d'un complexe bactériolytique pas plus tard que 3 h après l'infection.

3. Complexe bactériolytique selon la revendication 1, qui est approprié pour être appliqué à un emplacement supposé d'une pénétration pathogène par un bandage (papier tissé) imprégné du complexe bactériolytique.

4. Complexe bactériolytique selon la revendication 1, grâce auquel un aérosol contenant le complexe bactériolytique est approprié pour traiter un foyer d'infection probable.

5. Complexe bactériolytique selon la revendication 1, grâce auquel le complexe bactériolytique est approprié pour traiter la forme cutanée de l'anthrax par injections sous-cutanées, comprenant des injections ou un traitement par aérosol du foyer de lésion, et pour traiter les formes généralisées par injections sous-cutanées.

6. Complexe bactériolytique selon l'une quelconque des revendications 1 à 5, grâce auquel le complexe bactériolytique est utilisé sous la forme d'une solution dans un tampon phosphate qui est préparée immédiatement avant l'application.

7. Complexe bactériolytique selon la revendication 2, grâce auquel la solution de complexe bactériolytique est appropriée pour être introduite par voie sous-cutanée dans l'extrémité où le nid d'infection est situé.

8. Complexe bactériolytique selon la revendication 1, grâce auquel le complexe bactériolytique est approprié pour être introduit pas plus tard que 3 h ou 24 h après l'infection.

9. Composition pharmaceutique comprenant un complexe bactériolytique selon l'une quelconque des revendications 1 à 8 destinée à une utilisation dans le traitement ou la prévention de l'anthrax.

10. Utilisation d'un complexe bactériolytique selon l'une quelconque des revendications 1 à 8 dans la fabrication d'un médicament pour le traitement et la prévention de l'anthrax.

11. Complexe bactériolytique produit par une souche bactérienne de *Lysobacter sp. XL 1* selon l'une quelconque des revendications 1 à 8 destiné à une utilisation en tant qu'un agent bactériolytique sur des cellules végétatives ou sporales de *Bacillus anthracis.*
